Europäisches Patentamt

European Patent Office

Office européen des brevets

(11) Veröffentlichungsnummer: **0 307 599 B1**

# (12) EUROPÄISCHE PATENTSCHRIFT

(45) Veröffentlichungstag der Patentschrift: **03.06.92**

(21) Anmeldenummer: **88112491.1**

(22) Anmeldetag: **01.08.88**

(51) Int. Cl.⁵: **A61K 31/245**, A61K 9/20, A61K 47/00

(54) **Arzneimittel für den Bereich der Mundhöhle.**

(30) Priorität: **12.08.87 DE 3726797**

(43) Veröffentlichungstag der Anmeldung:
**22.03.89 Patentblatt 89/12**

(45) Bekanntmachung des Hinweises auf die
Patenterteilung:
**03.06.92 Patentblatt 92/23**

(84) Benannte Vertragsstaaten:
**AT BE CH DE ES FR GB IT LI NL SE**

(56) Entgegenhaltungen:
**EP-A- 0 130 524**

**CHEMICAL ABSTRACTS, Band 76, Nr. 1, 3.
Januar 1972, Seite 31, Zusammenfassung Nr.
277q, Columbus, Ohio, US; A.D. INGLOT et
al.: "Topical treatment of cutaneous herpes
simplex in humans with the non-steroid antiinflammatory drugs mefenamic acid and indomethacin in demethylsulfoxide", & ARCH.
IMMUNOL. THER. EXP. 1971, 19(4) 555-66**

(73) Patentinhaber: **BAYER AG**

**W-5090 Leverkusen 1 Bayerwerk(DE)**

(72) Erfinder: **Leusner, Bernd, Dr.
Grüner Weg 148
W-5090 Leverkusen 1(DE)**
Erfinder: **Heiss, Rüdiger, Dr.
Heide 60
W-4155 Grefrath 1(DE)**
Erfinder: **Pelster, Bernd, Dr.
Pleisufer 6a
W-5205 St. Augustin(DE)**
Erfinder: **Fischer, Wolfgang, Dr.
August-Kierspel-Strasse 139
W-5060 Bergisch Gladbach 2(DE)**
Erfinder: **Ohm, Ulrich, Dr.
Altenberger-Dom-Strasse 20
W-5068 Odenthal(DE)**

**Beschreibung**

Die Erfindung betrifft die Verwendung von Etofenamat für die Herstellung von Arzneimitteln zur Behandlung von bakteriellen, viralen oder durch Pilze verursachten Entzündungen im Bereich der Mundhöhle.

Etofenamat (INN) (2-(2-Hydroxyethoxy)-ethyl-N-($\alpha,\alpha,\alpha$-trifluor-m-tolyl)-anthranilat) ist ein bekannter Wirkstoff mit stark antiphlogistischer Aktivität (Arzneim. Forschung 27 (I), 6b (1977) 1326-1333). Etofenamat wird als Wirkstoff zur Behandlung von traumatisch bedingten Entzündungen, beispielsweise nach Druck- oder Stoßverletzungen, eingesetzt. Zu dieser Gruppe von Entzündungen gehören auch rheumatisch bedingte Entzündungen an Gelenken und Weichteilen. Arzneimittel, die Etofenamat enthalten, sind z.B. aus der EP-A-0130524 bekannt.

Etofenamat enthaltende Arzneimittel liegen in der Regel als Gel oder als Creme vor, die auf die trockene Haut aufgetragen werden. Gele enthalten als Adjuvantien z. B. Polyacrylsäuren, Wasser und Isopropanol (DAB 9, Ausg. 1986, S. 1186); für nichttopische Anwendungen sind diese Gele und Cremes nicht geeignet.

Es wurde die Verwendung von Etofenamat für die Herstellung von Arzneimitteln zur Behandlung von bakteriellen, viralen oder durch Pilze verursachte Entzündungen im Bereich der Mundhöhle gefunden.

Es ist überraschend, daß Etofenamat außer bei traumatisch bedingten Entzündungen auch bei bakteriellen, viralen oder durch Pilze verursachten Entzündungen eingesetzt werden kann.

Dies trifft insbesondere für bakteriell, viral oder durch Pilze bedingte Entzündungen der Mundschleimhäute zu. Arzneimittel für den Bereich der Mundhöhle müssen eine gute Haftung auf dem Gewebe unter den Bedingungen des Mundmilieus, eine hohe Stabilität, eine hohe Liberationsrate und eine hohe Akzeptanz beim Patienten aufweisen. Zubereitungen von Etofenamat, die diesen Zweck erfüllen, sind bisher nicht bekannt geworden. Die erfindungsgemäße Verwendung der Arzneimittel erfüllt in hohem Maß diese Anforderungen und ist daher als ein Fortschritt der Technik zu bezeichnen.

Bakteriell oder viral bedingte Entzündungen im Bereich der Mundhöhle können z.B. Pulpitis, Gingivitis, Parodontitis marginalis und apicalis, Dentitio difficilis, Stomatitis und Aphthen sein. Als Beispiel für eine Pilzinfektion der Mundschleimhaut sei hier die Candidiasis (Soor) genannt. Bevorzugt werden die erfindungsgemäßen Arzneimittel zur Behandlung von Gingivitis und Parodontitis eingesetzt. Weiterhin finden die Arzneimittel Anwendung bei der Behandlung von prothesenbedingten Schleimhautläsionen sowie der Wundbehandlung nach zahnärztlich chirurgischen, kieferchirurgischen und parodontalchirurgischen Eingriffen.

Die erfindungsgemäßen Arzneimittel enthalten Etofenamat im allgemeinen bis zu 20 Gewichtsprozent, bevorzugt zu 1 bis 10 Gewichtsprozent bezogen auf die Zubereitungsform. Insbesondere bevorzugt sind Zubereitungen mit 2 bis 8 Gewichtsprozent Etofenamat.

Selbstverständlich ist es möglich, daß die erfindungsgemäßen Arzneimittel außer Etofenamat auch andere Wirkstoffe oder wirksame Hilfsstoffe enthalten. Beispielsweise seien genannt:

Lokalanästhetika (z. B. Tetracain, Benzocain, Procain, Lidocain und deren Salze)

Chemotherapeutika (z. B. Sulfanilamid)

Antibiotika (z. B. Neomycin, Tyrothricin, Chlor-Tetracyclinhydrochlorid)

Antimykotika (z.B. Clotrimazol, Bifonazol, Nystatin, Miconazol, Amphotericin B)

Desinfektionsmittel (z. B. Benzalkoniumchlorid, Dequaliumchlorid, Chinosol, Chlorhexidindigluconat, Thymol)

Pflanzenextrakte (z. B. Chamazulen, Extr. Flor. Chamomillae)

Adstringentien (z. B. Tannin, Aluminiumchlorid)

Die erfindungsgemäßen Arzneimittel können als halbfeste oder flüssige Zubereitungen vorliegen. Flüssige Zubereitungen können beispielsweise in Form von Mundwässern,

Tinkturen oder Aerosolen formuliert werden. Halbfeste Arzneimittel können Salben, Cremes, Gele, Emulsionen oder Pasten sein. Die Arzneimittel können auch Wundverbänden, wie sie z. B. nach parodontalchirurgischen Eingriffen verwendet werden, zugesetzt werden. Ebenso ist ein Zusatz der Arzneimittel zu Prothesenhaftmitteln (z.B. für Immediat- und Interimsprothesen und bei Prothesenstomatitis) möglich. Weiterhin können die Arzneimittel in medikamentösen Einlagen zur Pulpitistherapie (indirekte oder direkte Überkappung) und bei endodontischen Behandlungen verwendet werden. In einer besonderen Ausführungsform der vorliegenden Erfindung liegt Etofenamat in Form einer Haftemulsion vor.

Komponenten für die erfindungsgemäße Haftemulsion sind hydrophile Hilfsstoffe. Hierbei handelt es sich um rein natürliche, halb- oder vollsynthetische Polymere, welche neben ihrem Quellvermögen eine gute Haftfähigkeit auf der Mundschleimhaut erzielen.

Als Beispiele für natürliche Polymere seien genannt:

| | |
|---|---|
| Gelatine: | Gelatine ist ein gereinigtes Protein, das entweder durch partielle saure (Typ A) oder alkalische Hydrolyse (Typ B) von tierischem Kollagen gewonnen wird. Die Substanz kann auch aus einer Mischung der beiden Typen bestehen. |
| Pektin: | Pektine bestehen im wesentlichen aus Ketten von 1,4-$\alpha$-glykosidisch verbundenen Galakturonsäure-Einheiten, deren Säuregruppen zu 20 bis 70 % mit Methanol verestert sind. Die Pektine bilden langgestreckte Makromoleküle, die auch noch etwas Galaktose und Arabinose enthalten und schwachsaure Eigenschaften aufweisen. Bevorzugt sind die Pektine aus Citrus vom Molekulargewicht ca. 150.000 bis 300.000. |
| Alginsäure bzw. Natriumalginat: | Aus Braunalgen gewonnene Polymannuronsäure bzw. deren Alkalisalze z.B. Natrium- oder Kaliumsalz sowie Ammoniumsalz. |
| Arabisches Gummi: | Arabisches Gummi ist eine an der Luft erhärtete gummiartige Ausscheidung, die auf natürliche Weise oder nach Einschneiden des Stammes und der Zweige von Acacia senegal L. Willdenow oder anderer afrikanischer Acacia-Arten austritt. |
| Traganth: | Traganth ist die an der Luft erhärtete gummiartige Ausscheidung, die natürliche oder nach Einschneiden aus Stamm und Ästen von Astragalus gummifer Labillardere und von bestimmten anderen westasiatischen Arten der Gattung Astragalus ausfließt. |
| Galaktomannan: | Galaktomannan ist ein Polymer aus Mannose und Galaktose. |
| Karayagummi: | Karayagummi ist die an der Luft erhärtete gummiartige Ausscheidung, die natürlich oder nach Einschneiden aus Stamm und Ästen von Sterculia-Arten ausfließt. |

Als Beispiele für halbsynthetische Polymere seien genannt:

Celluloseäther wie Methyl-, Methylhydroxyethyl-, Hydroxymethyl-, Ethyl-, Propyl- oder Hydroxypropylmethylcellulose oder Carboxymethylcellulose bzw. dessen Natriumsalz.

Als Beispiele für synthetische Polymere seien genannt:

Polymethacrylsäure bzw. deren Salze

Polymethylmethacrylsäure bzw. deren Salze

Polyvinylpyrrolidon

Es ist selbstverständlich möglich, diese Komponenten zu kombinieren. Dabei ist der Zusatz von Alkoholen und/oder Wasser sowie gegebenenfalls Geschmacks-, Konservierungs-und Farbstoffen möglich.

Alkohole sind im wesentlichen niedere Alkohole, bevorzugt Ethanol.

Die erfindungsgemäßen Haftemulsionen enthalten im allgemeinen

Etofenamat    zu 1 bis 10 Gew.-Teilen,

Quellmittel    zu 1 bis 20 Gew.-Teilen,

Wasser    zu 60 bis 90 Gew.-Teilen,

und gegebenenfalls Geschmacksstoffe, wie Menthol, Konservierungsmittel und/oder Farbstoffe.

Es wurde auch ein Verfahren zur Herstellung von Etofenamat enthaltenen Arzneimitteln zur Behandlung von durch Bakterien, Viren oder Pilzen verursachten Entzündungen im Bereich der Mundhöhle gefunden, das dadurch gekennzeichnet ist, daß Etofenamat mit den flüssigen Komponenten gemischt, bei 30 bis 70 Grad Celsius homogenisiert wird und anschließend unter Rühren die festen Komponenten eingetragen werden.

Die Homogenisierung erfolgt im allgemeinen bei 30 bis 70 ° C, bevorzugt bei etwa 50 ° C.

Die festen Komponenten sind im wesentlichen die oben genannten Hilfsstoffe. Flüssige Komponenten sind im wesentlichen die oben genannten Alkohole und Wasser.

Die pastöse Masse wird noch einige Stunden bis zum völligen Ausquellen weitergerührt und nach dem Abkühlen gegebenenfalls mit Geschmacksstoffen versetzt.

Die erhaltene Emulsion ist ohne Zusatz von Emulgatoren in dieser Zusammensetzung stabil.

Beispiele 1 bis 20   (Herstellung):

<u>Beispiele für Etofenamat-Zubereitungen:</u>

(Menge der Komponenten in mg pro g Zubereitung)

| Beispiel Nr. | | 1 | 2 | 3 | 4 | 5 | 6 | 7 | 8 | 9 | 10 | 11 | 12 |
|---|---|---|---|---|---|---|---|---|---|---|---|---|---|
| Etofenamat | | 25 | 50 | 50 | 50 | 50 | 100 | 50 | 50 | 25 | 25 | 25 | 25 |
| Wasser | | 882 | 460 | 475 | 470 | 470 | 450 | - | - | - | 325 | 325 | 662 |
| Benzylalkohol | | 15 | - | - | - | - | - | - | - | - | - | - | - |
| Ethanol 96 % | | - | 450 | 450 | 450 | 450 | 450 | - | - | - | - | - | - |
| NaOH 2n | | - | - | - | - | - | - | - | - | - | 450 | 450 | 180 |
| Pektin | | - | - | - | - | - | - | - | 160 | 160 | - | - | - |
| Klucel® HF | 1) | - | - | - | - | 24 | - | - | - | - | - | - | - |
| Meyprogat® T 100 | 2) | 70 | - | - | - | - | - | - | - | - | - | - | - |
| Tylose® MH 300 P | 3) | - | 40 | - | - | - | - | - | - | - | - | - | - |
| Tylose® MH 4000 | 3) | - | - | 25 | - | - | - | - | - | - | - | - | - |
| Tylose® MH 1000 | 3) | - | - | - | 25 | - | - | - | - | - | - | - | - |

EP 0 307 599 B1

Beispiele 1 bis 20  (Herstellung):

<u>Beispiele für Etofenamat-Zubereitungen:</u>    (Fortsetzung)

(Menge der Komponenten in mg pro g Zubereitung)

| Beispiel Nr. | | 1 | 2 | 3 | 4 | 5 | 6 | 7 | 8 | 9 | 10 | 11 | 12 |
|---|---|---|---|---|---|---|---|---|---|---|---|---|---|
| Eudispert® | 4) | - | - | - | - | 6 | - | - | - | - | - | - | - |
| Eudragit® L 100 | 5) | - | - | - | - | - | - | - | - | - | 200 | - | - |
| Eudragit® L 100-55 | 6) | - | - | - | - | - | - | - | - | - | - | 200 | - |
| Eudragit® S 100 | 7) | - | - | - | - | - | - | - | - | - | - | - | 133 |
| Gelatine | | - | - | - | - | - | - | - | 160 | 160 | - | - | - |
| Plastibase® | 8) | - | - | - | - | - | - | - | 470 | 487 | - | - | - |
| Natriumcarboxy-methylcellulose | | - | - | - | - | - | - | - | 160 | 160 | - | - | - |
| Menthol | | 8 | - | - | - | - | - | - | - | 8 | - | - | - |

1) Klucel® HF ist eine Hydroxypropylcellulose (Hercules Filter Corp.)
2) Meyprogat® T 100 ist ein Galaktomannan (Meyhall Chemical GmbH)
3) Tylose® MH 300 P, 4.000, 1.000 sind Methylcellulosen bzw. Methylhydroxyethylcellulosen (Hoechst AG)
4) Eudispert® ist ein Copolymer  aus Methylmethacrylat und Methacrylsäure (Röhm Pharma
   GmbH)
5)⎫
6)⎬ Eudragit L 100, L 100-55, S 100 sind Copolymerisate auf Basis von Methacrylsäure und
7)⎭ Methacrylsäuremethylester (Röhm Pharma GmbH)
8) Plastibase® besteht aus Polyethylen und Paraffinöl (E. R. Squibb & Sons, Inc.)

EP 0 307 599 B1

Beispiele 1 bis 20 (Herstellung):

Beispiele für Etofenamat-Zubereitungen:

(Menge der Komponenten in mg pro g Zubereitung)

| Beispiel Nr. | 13 | 14 | 15 | 16 | 17 | 18 | 19 | 20 |
|---|---|---|---|---|---|---|---|---|
| Etofenamat | 10 | 25 | 50 | 100 | 50 | 10 | 25 | 50 |
| Wasser | 867 | 852 | 827 | 777 | 857 | 515 | 500 | 475 |
| Benzylalkohol | 15 | 15 | 15 | 15 | 15 | - | - | - |
| Ethanol 96 % | - | - | - | - | - | 450 | 450 | 450 |
| Pektin | 100 | 100 | 100 | 100 | 70 | - | - | - |
| Klucel® HF 9) | - | - | - | - | - | 25 | 25 | 25 |
| Menthol | 8 | 8 | 8 | 8 | 8 | - | - | - |

9) Klucel® HF ist eine Hydroxypropylcellulose (Hercules Filter Corp.)

Beispiel 21 (Anwendung)

1. Tierexperimenteller Wirksamkeitsnachweis:

a) Prüfsubstanz

Es wurde eine 2,5-gew.-%ige ethanolische Lösung (Grundlage) von Etofenamat eingesetzt.

b) Versuchsaufbau:

In Anlehnung an das in Caries Res. 19, 516 - 518 (1985) publizierte Verfahren wurde an 6 Gruppen von je 14 Wistar-Ratten (7 ♂, 7 ♀) durch Fütterung einer speziellen Diät (2.000 M) unter Zugabe von 1 % Rattenhaaren eine Parodontitis induziert. Die Hälfte der Tiere (3 Gruppen) wurde vor Versuchsbeginn durch orale Applikation einer Keimsuspension von Actinomyces viscosus Nyl infiziert, um die Progredienz der Parodontitis zu steigern.

Für den 9 Wochen dauernden Versuch wurden die Gruppen wie folgt aufgeteilt:

```
Gruppe 1  (ohne A. viscosus) ⟩  keine Therapie
Gruppe 2  (mit        "      ) ╱

Gruppe 3  (ohne       "      ) ⟩  Applikation der
Gruppe 4  (mit        "      ) ╱  Grundlage

Gruppe 5  (ohne       "      ) ⟩  Behandlung mit
Gruppe 6  (mit        "      ) ╱  Etofenamat +
                             )    Grundlage
```

c) Versuchsdurchführung:

Die Versuchstiere der Gruppen 5 und 6 erhielten 3 x 0,08 ml Etofenamatlösung pro Tag im Abstand von 8 Stunden, entsprechend einer Wirkstoff-Tagesdosis von 60 mg/kg KG bezogen auf ein Anfangsgewicht von 100 g. Die Wirksubstanz wurde mittels einer Tuberkulinspritze (ohne Nadel) direkt in die Mundhöhle der Tiere appliziert. Auf diese Weise wurde auch den Tieren der Gruppen 3 und 4 die wirkstofffreie Grundlage in der gleichen Frequenz und Menge (3 x 0,08 ml/Tag) verabreicht.

d) Versuchsergebnisse:

Im Anschluß an den Versuch wurden die Tiere untersucht. Nach der Methode in Arch. Oral Biol. 27, 651-658 (1987) wurde der alveolare Knochenschwund als Parameter für das Ausmaß der parodontalen Entzündung bestimmt. Dabei zeigte sich, daß die mit Wirksubstanz behandelten Tiere statistisch signifikant geringeren parodontalen Knochenabbau aufwiesen als die Tiere, die nicht behandelt wurden oder nur die Grundlage erhielten.

**Patentansprüche**

1. Verwendung von Etofenamat zur Herstellung von Arzneimitteln zur Behandlung von bakteriellen, viralen oder durch Pilze verursachten Entzündungen im Bereich der Mundhöhle.

2. Verwendung nach Anspruch 1, dadurch gekennzeichnet, daß es sich bei den Arzneimitteln um Haftemulsionen handelt.

3. Verwendung nach Ansprüchen 1 und 2, dadurch gekennzeichnet, daß das Arzneimittel 0,5 bis 20 Gew.-% Etofenamat enthält.

4. Verwendung nach Ansprüchen 1 bis 3, dadurch gekennzeichnet, daß das Arzneimittel Etofenamat, eine oder mehrere hydrophile Hilfsstoffe, Wasser und einen oder mehrere Alkohole enthält.

**Claims**

1. Use of etofenamate for the preparation of medicaments for the treatment of inflammations in the region of the oral cavity which are bacterial, viral or caused by fungi.

2. Use according to Claim 1, characterised in that the medicaments are adhesive emulsions.

3. Use according to Claims 1 and 2, characterised in that the medicament contains 0.5 to 20% by weight of etofenamate.

**4.** Use according to Claims 1 to 3, characterised in that the medicament contains etofenamate, one or more hydrophilic auxiliaries, water and one or more alcohols.

**Revendications**

**1.** Utilisation d'étofénamate pour la préparation de médicaments pour le traitement d'inflammations d'origine bactérienne, d'origine virale ou provoquées par des champignons, dans la région de la cavité buccale.

**2.** Utilisation selon la revendication 1, **caractérisée en ce que,** quant aux médicaments, il s'agit d'emulsions adhésives.

**3.** Utilisation selon les revendications 1 et 2, **caractérisée en ce que** le médicament contient de l'étofénamate à raison de 0,5 à 20% en poids.

**4.** Utilisation selon les revendications 1 à 3, **caractérisée en ce que** le médicament contient de l'étofénamate, une ou plusieurs substances auxiliaires hydrophiles, de l'eau, ainsi qu'un ou plusieurs alcools.